(19) Europäisches Patentamt
European Patent Office
Office européen des brevets

(11) Publication number: 0 134 483
A2

# (12) EUROPEAN PATENT APPLICATION

(21) Application number: 84107948.6

(22) Date of filing: 06.07.84

(51) Int. Cl.⁴: A 61 K 7/48

(30) Priority: 08.07.83 US 511816

(43) Date of publication of application:
20.03.85 Bulletin 85/12

(84) Designated Contracting States:
AT BE CH DE FR GB IT LI LU NL SE

(71) Applicant: S.C. JOHNSON & SON, INC.
1525 Howe Street
Racine, Wisconsin 53403(US)

(72) Inventor: Muoio, Erland L.
18 Sandalwood Court
Racine Wisconsin, 53402(US)

(74) Representative: Baillie, Iain Cameron et al,
c/o Ladas & Parry Isartorplatz 5
D-8000 München 2(DE)

## (54) Skin care composition.

(57) An aqueous, stable, non-occlusive composition for personal use includes an emollient base, a carboxyl vinyl polymer thickener and an amphoteric emulsifier.

EP 0 134 483 A2

# SKIN CARE COMPOSITION

## BACKGROUND OF THE INVENTION

### Field of the Invention

The present invention relates to an improved, topically applied personal care composition. In particular, it relates to a stable, non-occlusive formulation for imparting enhanced skin care properties.

### Description of The Prior Art

It has long been desired to provide skin conditioning without the heavy, greasy and occlusive characteristics of conventional vaseline-,mineral oil- and lanolin-based compositions. Skin and scalp conditioning is recognized as a necessary adjunct to reduce or prevent harmful drying effects to the skin and hair caused by use of detergents, toilet bar soaps, shampoos, antiperspirants and the like. It is also well understood that the drying and cracking effects on the skin caused by extended exposure to the sun and low humidity requires an effective conditioner and moisturizer to return cutaneous tissue to its normal softness and moisture levels.

It is now understood that from a biochemical standpoint, dryness is, in part, a measure of the water content of the skin. The skin becomes dry because of excessive loss of water from its surface and the subsequent loss of water from the stratum corneum.

Continuous and prolonged immersion in various soaps and detergents, use of shampoos and antiperspirants and exposure to the sun or infrared and/or ultraviolet radiation all contribute to drying the stratum corneum. Surfactant based cleansers, including soaps and shampoos, promote dissolution of skin surface lipids such as fatty acids, horny layer lipids and hydroscopic water-soluble components in the corneum. This, in turn, leads to the development of so called "dry skin" with concomitant cracking, flaking and scaling of dead corneocytes.

It has long been a desideratum of the personal care industry to find a composition with a non-oily feel which would improve skin condition by providing extended therapeutic benefits; by aiding in healing chapped and cracked skin; by protecting against excess cutaneous dryness and by promoting long term moisturizing, without sacrificing cosmetic elegance. Cosmetic elegance is understood to mean imparting to the skin a sensation of smoothness and dryness rather than oiliness or greasiness, free from chemical irritation.

For this and other purposes attempts have been made to emulsify mineral oil or petrolatum to provide a beneficial skin care composition. However, to provide a suitable dispersion, high levels of anionic emulsifiers have generally proven necessary. At high levels, anionic emulsifiers tend to irritate the skin by neutralizing skin fatty acids and shifting the skin pH from its slightly acid level to alkaline levels. In addition, such emulsifiers tend to unduly lower viscosity levels and fail to provide satisfactory stable emulsions.

## SUMMARY OF THE INVENTION

It is an object of the invention to provide a personal care composition having enhanced skin conditioning properties with acceptable cosmetic elegance.

It is another object to provide an emollient formulation having acceptable viscosity and stability levels.

These and other objects are attained in an aqueous, stable, non-occlusive, personal care composition comprising an emollient base, a carboxyl vinyl polymer thickener and an amphoteric emulsifier. The thickener is employed in amounts sufficient to permit a controlled topical application of the composition. The emulsifier is employed in amounts sufficient to control the pH of the composition at a level compatible with topical skin application, usually from 4.5 to 6, and to stabilize the composition, which is an oil-in-water emulsion. For this and other purposes an emulsifier having an isoelectric range pH from about 1-5 is preferably employed. The amphoteric emulsifier or surfactant is preferably an alkaline salt of (i) a N-fatty aminopropionate or (ii) a N-fatty iminodipropionate or (iii) mixtures thereof.

It has been found that the amphoteric surfactant at relatively low concentration levels can emulsify relatively high solids concentrations of active skin care ingredients, such as emollients, to provide longer lasting moisturization, enhanced protection against dryness, healing of chapped and cracked skin and a smoother skin feel. Use of the amphoteric emulsifier also results in

the formation of a better dispersed emollient of a relatively small particle size. Better dispersions provide more stable emulsions. That stability property is especially important when mineral oil, petrolatum or other greasy conditioning emollients or moisturizers are employed. Generally, emulsifiers, especially anionic surfactants, tend to irritate the skin when employed in conventional amounts for dispersing oily emollients. It is a feature of the present invention that because of the lower amounts of emulsifier employed, the skin remains generally free of such irritation.

In order to provide appropriate flowability characteristics to the inventive compositions it has been found that a thickener is required. Carboxy vinyl polymer resins are suitable to provide proper viscosity control. In addition they assist in providing proper pH control when utilized together with the amphoteric emulsifier.

The ambient pH of subcutaneous tissue is a function of the fatty acids present on the skin and is generally from 4.5 to 6.0. The amphoteric emulsifiers of the invention exhibit a highly alkaline solution pH, preferably on the order of pH 12. The emulsifiers preferably possess an isoelectric range pH within the range from 1-5. The carboxyl vinyl polymer thickeners exhibit an acidic solution pH usually less than about 3. Accordingly, during formulation of the composition of the invention, the basic groups on the emulsifier neutralize the pendant acid groups on the thickener and the solution pH of the composition can be adjusted to approach that of the skin to which it is applied, usually from 5 to 5.5. Therefore, skin fatty acids which have been saponified

after treatment with alkaline soaps and the like [illegible] regenerated and converted to the free acid form to further condition and protect the skin. In addition, the solubility of the amphoteric emulsifier is enhanced when the final solution pH is above the isoelectric range pH. That enables a more uniform dispersion to be formed. The aqueous composition is preferably applied as a lotion, milky emulsion, cream or paste. Unless otherwise noted all weights are based on the total weight of the composition.

## DESCRIPTION OF PREFERRED EMBODIMENTS

The amphoteric emulsifier of the invention has an alkaline solution pH and is adapted to neutralize the carboxyl groups of the thickener to adjust solution viscosity and solution pH. The emulsifier preferably has an isoelectric pH range from pH 1-5, with an isoelectric point from 3.0 to 4.0. For this and other purposes amphoteric emulsifiers are employed containing as the basic center, an amine group and, as an acidic center, a carboxyl group or sulfonic group. Typical amphoterics include the betaines, as the N-alkylbetaines, the N-alkylsulphobetaines and the N-alkylamidobetaines, cycloimidinium compounds, as the alkylimidazolines, and the derivatives of asparagine, such as the N,N-dialkylaminoalkyl-N-2-alkyl (fatty) asparagines.

Examples of typical betaines includes the propylamido betaines derived from $C_{10}$-$C_{16}$ fatty acids, their corresponding propylamido sultaines and $C_{10}$-$C_{16}$ alkyl sultaines, wherein the cationic and anionic groups are separated by a propylene group and the remaining

groups are methyls. Examples of other such usable emulsifiers include 4-[N-coconutacylamido-propylene-N,N-di(2 hydroxy-propyl)-ammonio]butane-1-sulfonate; 2[N-pentadecylamido-propylene-N-(3-hydroxypropyl)-N-propyl-ammonio]ethane-1-sulfonate; 4(N-laurylmorpholino)-2-hydroxybutanoate; 3-(N-laurylmorpholino)-propane-1-sulfonate; 3-(N-tridecyl-N-methyl-N-propyl)-aminopropanoate; 4-(N,N,N-trimethyl-ammonio)stearate; 3-(N-methyl-N-(2-hydroxyethyl)-N-propylammonio]eicosane-1-sulfonate; 5-[N,N-(3-hydroxy-propyl)-N-methylammonio]-3-hydroxydocosane-1-carboxylate; N-coconutalkyl betaine; C-cetyl betaine; C-hexadecyl betaine; 3-(N,N-dimethyl-N-coconutalkyl-ammonio)-2-hydroxypropane-1-sulfonate; 6-coconutacylamido-3-trimethylammoniohexanoate; 7-coconutacylamido-4-tri(2-hydroxyethyl)-heptane-1-sulfonate; 3-[N-(3-coconutacyl-amidopropyl)-N,N-dimethylammonio]-propane-1-sulfonate; 3-[N-(3-coconutacylamidopropyl)-N,N-di-(2-hydroxyethyl)ammonio]-2-hydroxypropane-1-sulfonate; 6-(N-coconutalkyl-N,N-dimethyl)hexanoate; 5-(N,N-dipropyl-N-dodecylammonio)pentane-1-sulfonate; 3-(N-methylmorpholino)-stearate.

The preferred amphoteric emulsifier of the invention is a water-soluble, N-substituted amino acid derivative, a beta-alanine. Such amino acids are derived by the condensation of fatty primary amines and acrylic monomers. The reaction is selectively controlled to provide either a N-fatty aminopropionate-$RCH_2NHCH_2CH_2COOM$ or a N-fatty imino propionate $RCH_2N(CH_2CH_2COOM)_2$, where N is a salt forming cation or hydrogen and R is an alkyl group having from about 8 to 18 carbon atoms. The salt forming cation is preferably an alkaline cation.

In acidic solution the preferred amphoterics exist as cationic amine salts, while in alkaline solution they are anionic carboxylates. In the isoelectric range from 1.3 to 5.0, the amphoteric exists predominantly as a zwitterion with positive and negative tails. In the isoelectric range the zwitterion exhibits minimum solubility, as well as reduced foaming, detergency and wetting. Accordingly, it is preferred to employ the amphoteric at a solution pH between about 5.0 and 5.5 to enhance solubility and wetting and to provide a compatible skin pH.

The most preferred amphoterics are known commercially as the Deriphats, and include the alkali salts of N-($C_8$-$C_{18}$) fatty, ß-aminopropionate or ß-iminodipropionate having a typical solution pH on the order of 12. Especially preferred emulsifiers providing particularly enhanced stable dispersions include sodium-N-coco-ß-aminopropionate; disodium-N-tallow-ß-imino-dipropionate and disodium N-lauryl-ß-iminodipropionate.

The carboxy vinyl polymer thickener of the invention is an acid resin which contributes to the viscosity and stability of the emulsion and in combination with the emulsifier acts to provide a solution pH in the range suitable for non-irritating topical application. Upon neutralization with an anionic moiety as the amphoteric emulsifier of the invention, the thickener's neighboring carboxyl groups repel each other and uncoil and extend the polymeric chain. The extended chains of the thickener interfere with each other and hinder the movements of neighboring chains. The fluid containing the thickener, then exhibits increased viscosity.

The carboxyl vinyl polymer of the invention can include crosslinked graft copolymers having (a) at least one non-ionic monomer, (b) at least one unsaturated carboxylic monomer, (c) polyethylene glycol and (d) a crosslinking agent comprising ethylene glycol dimethacrylate, diallyl phthalate, the divinylbenzenes and polyallylsucroses.

The polyethylene glycol (c) used generally has a molecular weight of 200 to several million and preferably 300 to 30,000.

The non-ionic monomers (a) can be those such as: vinyl acetate, vinyl stearate, vinyl laurate, vinyl propionate, allyl stearate, allyl laurate, diethyl maleate, allyl acetate, methyl methacrylate, cetyl vinyl ether, stearyl vinyl ether and 1-hexene.

The ionic monomers (b) are unsaturated carboxylates such as: crotonic acid, allyloxyacetic acid, vinylacetic acid, maleic acid, acrylic acid and methacrylic acid.

The crosslinked graft copolymers which can be used according to the invention preferably comprise: (a) from 5 to 85% by weight of at least one non-ionic monomer; (b) from 3 to 80% by weight of at least one ionic monomer; (c) from 2 to 50% by weight, but preferably from 5 to 30%, of polyethylene glycol, and (d) from 0.01% to 8% by weight of a crosslinking agent, the percentage of the crosslinking agent being expressed relative to the total weight of (a)+(b)+(c). Such crosslinked graft copolymers generally have a molecular weight of 10,000 to 1,000,000 and preferably of 15,000 to 500,000.

The carboxyl vinyl thickeners can also include the tetra- and penta-polymers which comprise the copolymers resulting from the copolymerization of:

(a) an unsaturated acid which is crotonic acid or allyloxyacetic acid; (b) vinyl acetate or vinyl propionate; (c) at least one branched allyl or methallyl ester and (d) a monomer taken from the group comprising: (i) a vinyl ether of the formula:

$$CH_2=CH-O-R_3$$

in which $R_3$ is a linear or branched alkyl radical having 1 to 12 carbon atoms, and preferably methyl, ethyl, isopropyl, t-butyl, octyl or dodecyl, (ii) a fatty chain vinyl ester of the formula:

$$R_4-\underset{\underset{O}{\|}}{C}-O-CH=CH_2$$

in which $R_4$ is a linear alkyl radical having 7 to 11 carbon atoms, and preferably octyl, decyl or dodecyl, and (iii) a linear allyl or methallyl ester of the formula:

$$R_5-\underset{\underset{O}{\|}}{C}-O-CH_2-\underset{\underset{R'}{|}}{C}=CH_2$$

in which R' is a hydrogen atom or a $-CH_3$ radical and $R_5$ is a linear alkyl radical having 1 to 11 carbon atoms, and preferably methyl, heptyl, nonyl or undecyl.

The abovementioned copolymers result, in particular, from the copolymerization of 2-15% and preferably 4-12% of the unsaturated acid, 55-89.5% and preferably 65-85% of the vinyl ester, 8-20% and preferably 10-17% of at least one allyl or methallyl ester, and 0.5-10% and preferably 1-6% of a monomer taken from the group comprising: a vinyl ether, a vinyl ester and an allyl or methallyl ester as noted above as (i), (ii) and (iii).

In one particular embodiment, these copolymers are cross-linked with the aid of a crosslinking agent in an amount from 0.1 to 1.2% by weight.

Other suitable thickeners include the ter-, tetra- and penta-polymers which result from the copolymerization of (a) at least one water-insoluble monomer of the formula:

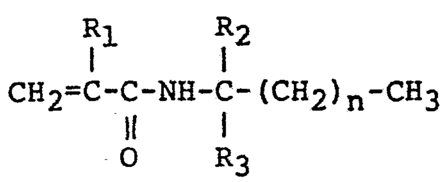

in which $R_1$, $R_2$ and $R_3$ represent either a hydrogen atom or a methyl radical and n is 0 or an integer from 1 to 10, inclusive, and preferably N-(tertiary butyl)-acrylamide, N-octyl-acrylamide, N-decyl-acrylamide, N-dodecyl-acrylamide, N-[1-(1,1-dimethyl)propyl]-acrylamide, N-[1-(1,1-dimethyl)-acrylamide and N-[1-(1,1-dimethyl)-pentyl]-acrylamide, as well as the corresponding methacrylamides, and (b) at least one water-soluble monomer of the formula:

$$R_4-CH=\underset{\displaystyle R_5}{\underset{|}{C}}-(CH_2)_m-CO\overset{\displaystyle R'}{\overset{|}{N}}-Z-OH$$

in which R' represents a hydrogen atom or a methyl radical, Z represents a linear or branched alkylene radical having 1 to 6 carbon atoms, which is unsubstituted or substituted by one or two hydroxymethyl groups, and m is 0 or 1 and $R_4$ is H or $-COR_6$, $R_6$ being OH or $-NH-R_7$, $R_7$ denoting H or -Z-OH, and $R_5$ is H or $-CH_3$ when m = 0, and $R_4$ is H and $R_5$ is $CO-R_6$ when m = 1, and in particular the mono-, bis- or trihydroxy-alkyl-acrylamides or -methacrylamides, in which alkyl is a linear or branched group containing 1 to 6 carbon atoms, such as methyl, ethyl, propyl, butyl, 1,1-dimethyl-butyl or propyl; and N-mono- or -di-hydroxyalkyl-malic acid, N-mono- or -di-hydroxyalkyl-itaconanic acid and the corresponding amides, in which alkyl preferably is methyl, and (c) at least one further monomer taken from the group comprising acrylic acid and maleic anhydride.

Other suitable carboxy vinyl polymers include:

1. The non-crosslinked water-soluble polymers of acrylic acid or methacrylic acid or crotonic acid and copolymers of these monomers with a monoethylenically unsaturated monomer, such as ethylene, vinylbenezene, vinyl acetate, vinyl methyl ether and acrylamide and their water-soluble salts.

2. The terpolymers of vinyl acetate, crotonic acid and the vinyl ester of a saturated aliphatic monocarboxylic acid which is branched in the alpha-position and has at least 5 carbon atoms in the carboxylic radical.

These branches esters are derived, in particular, from an acid of the formula:

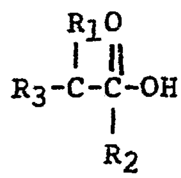

in which $R_1$ and $R_2$ are alkyl radicals and $R_3$ is H or an alkyl, alkaryl, aralkyl or aryl.

3. The tetrapolymers of the type described in French Pat. No. 2,062,801, which comprise about 70 to 80% of N-t-butylacrylamide or N-isopropylacrylamide, about 5 to 15% of acrylamide or methacrylamide, about 5 to 15% of N-vinylpyrrolidone and about 1 to 10% of acrylic acid or methacrylic acid, the percentages being by weight relative to the total weight of the composition.

The more preferred carboxy vinyl polymers include the hydrolyzed copolymers of ethylene and maleic anhydride monomers, copolymers of acrylic acid esters and copolymers of polyvinyl maleate and maleic anhydride.

The especially preferred polymers providing best results are the carbomers, which are homopolymers of acrylic acid cross-linked as described in U.S. 2,789,053,

issued July 2, 1957. The carbomers are polymers of acrylic acid which is cross-linked with about 0.75 to about 2% by weight of a polymer, preferably about 1%, of a co-polymerized polyalkenyl polyether. The polymerization of these monomers ordinarily is carried out in an inert hydrocarbon diluent with a free-radical catalyst. The preferred cross-linking polyalkenyl polyether monomers are polyallyl sucrose and polyallyl pentaerythritol, desirably containing an average of at least three allyl groups for each molecule of sucrose or pentaerythritol, the allyl groups attached thereto by means of ether linkages. The preferred polyallyl sucrose contains an average of 5 to 6 allyl groups per molecule of sucrose (theoretical maximum is 8 allyl groups) and the preferred polyallyl pentaerythritol contains 4 allyl ether groups per molecule (the theoretical maximum).

The carboxypolymethylenes embodied herein are high molecular weight polymers, e.g., usually having a molecular weight greater than 200,000 and preferably greater than 300,000. The most preferred range of molecular weight is between one million and five million. Forms of these resins may be obtained from B.F. Goodrich Company under the trademark "Carbopol," and in three different grades: Carbopol 934, Carbopol 940 and Carbopol 941. All are effective in this invention, although Carbopol 934 gives the most satisfactory compositions. The differences in the properties of 934, 940 and 941 are generally matters of degree, rather than kind, resulting in solution viscosities of somewhat differing values.

Other particularly preferred acrylic polymeric thickeners usable in the invention are a polyacrylic acid and its sodium and ammonium salts. Such polymers are

available under the marks Acrysol, Alcogum, North Thickener and Viscom, among others. Typical polyacrylic polymers have a structural formula:

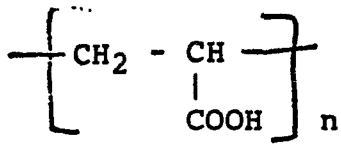

and exhibit molecular weights greater than 200,000, usually from 1,000,000 to 5,000,000.

The emollient of the invention can employ the broad range of cosmetic ingredients as conventionally utilized. Preferred emollients include:

(a) a humectant to aid in increasing emollence, reducing scaling, stimulating removal of built-up scale and improving the clarity of the film formed on skin during or after application. Typical humectants include polyhydric alcohols, polyalkylene glycols and more preferably alkylene polyols and their derivatives, including dipropylene glycol, polypropylene glycol, polyethylene glycol and derivatives thereof, sorbitol, hydroxypropyl sorbitol, hexylene glycol, 1,3-butylene glycol, 1, 2, 6-hexanetriol, ethoxylated glycerol, propoxylated glycerol and mixtures thereof. For best results the humectant is preferably propylene glycol.

(b) A hydrocarbon oil or wax including, for example, mineral oil, petrolatum, paraffin, ceresin, ozokerite, microcrystalline wax, polyethylene, and perhydrosqualene. The hydrocarbon oil or wax improves moisturizing effectiveness upon absorption into the outer skin layer (stratum corneum) to reduce transepidermal water loss. It also softens the skin and demonstrates other pharmacological benefits.

(c) An alkyl ester of a fatty acid having 10 to 20 carbon atoms. Methyl, isopropyl, and butyl esters of fatty acids are especially useful herein. Examples include hexyl laurate, isohexyl laurate, isohexyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate and most preferably, isopropyl palmitate. The alkyl esters improve skin feel and assist in uniformly spreading the oil phase of the emulsion during application of the composition of the invention.

(d) Fatty acids having 10 to 20 carbon atoms are employed to improve product viscosity and stability, and provide enhanced feel. Suitable examples include pelargonic, lauric, myristic, palmitic, stearic, isostearic, hydroxystearic, oleic, linoleic, ricinoleic, arachidic, behenic, and erucic acids.

(e) Fatty alcohols having 10 to 20 carbon atoms are utilized. Lauryl, myristyl, cetyl, hexadecyl, stearyl, isostearyl, hydroxystearyl, oleyl, ricinoleyl, behenyl, erucyl, and 2-octyl dodecanol alcohols are examples of satisfactory fatty alcohols.

Other types of emollients which can optionally be employed include:

1. A polyether derivative to reduce scaling, increase emollience and improve the clarity of the vehicle on application. For this and other purposes useful polyether derivatives include poly (ethylene oxide)

homopolymers, (molecular weight 100,000-5,000,000), poly (propylene oxide) homopolymers, polyoxypropylene derivatives of trimethylolpropane and other suitable poly (alkylene oxide) homo- and copolymers. Other polyether derivatives are the copolymers of polyethylene glycol and polypropylene glycol having a molecular weight from about 1000 to 2000, including polyoxyetylene/polyoxypropylene glycols.

2. Triglyceride esters, for example vegetable and animal fats and oils. Examples include caster oil, cocoa butter, safflower oil, cottonseed oil, corn oil, olive oil, cod liver oil, almond oil, avocado oil, palm oil, sesame oil, and soybean oil.

3. Acetoglyceride esters, such as acetylated monoglycerides.

4. Ethoxylated glycerides, such as ethoxylated glyceryl monostearate.

5. Alkenyl esters of fatty acids having 10 to 20 carbon atoms. Examples thereof include oleyl myristate, oleyl stearate, and oleyl oleate.

6. Fatty alcohol ethers such as ethoxylated fatty alcohols of 10 to 20 carbon atoms include the lauryl, cetyl, stearyl, isostearyl, oleyl, and cholesterol alcohols having attached thereto from 1 to 50 ethylene oxide groups or 1 to 50 propylene oxide groups.

7. Ether-esters such as fatty acid esters of ethoxylated fatty alcohols.

8. Lanolin and derivatives such as lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin alcohols, lanolin alcohols linoleate, lanolin alcohols ricinoleate, acetate of lanolin alcohols ricinoleate, ethoxylated hydrogenated lanolin, ethoxylated sorbitol lanolin and liquid and semisolid lanolin absorption bases.

9. Polyhydric alcohol esters, such as ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1, 3-butylene glycol monostearate, 1, 3-butylene glycol distearate, polyoxyethylene polyol fatty acid ester, sorbitan fatty acid esters, and polyoxyethylene sorbitan fatty acid esters.

10. Wax esters such as beeswax, spermaceti, myristyl myristate, stearyl stearate.

11. Beeswax derivatives, e.g. polyoxyethylene sorbitol beeswax. These are reaction products of beeswax with ethoxylated sorbitol of varying ethylene oxide content, forming a mixture of ether-esters.

12. Vegetable waxes including carnauba and candelilla waxes.

13. Phospholipids such as lecithin and derivatives.

14. Sterols such as cholesterol, cholesterol fatty acid esters are examples thereof.

15. Amides, such as fatty acid amides, ethoxylated fatty acid amides, solid fatty acid alkanolamides.

The amount of water or aqueous carrier to be included in the skin care compositions will vary, depending upon the desired consistency of the final product. By varying the amount of water, thickener and/or amphoteric surfactant present, it is possible to formulate a thick-flowing liquid or lotion, a semi-liquid thick cream, a paste, a stick, a gel, an alcoholic hydrogel, an emulsion, an alcoholic solution or a formulation suitable for use in an aerosol. In any event, the cosmetic composition should be provided in a form which can be uniformly spread on the skin. For this and other purposes, deionized water is preferred as the aqueous carrier.

The amphoteric emulsifier is employed in amounts sufficient to form a stable oil-in-water emulsion and to selectively control the pH of the composition at a level compatible with topical skin application. For this and other purposes the surfactant is generally employed in amounts from about 0.05 to 5%; preferably from 0.1 to 1% and, most preferably, in amounts from 0.2 to 0.6% by weight of the total composition.

The carboxyl vinyl polymer thickener is employed in amounts sufficient to provide the desired consistency or viscosity for controlled application and, in combination with the emulsifier, to selectively control the pH of the composition within a predetermined desired range. For this and other purposes the thickener is generally employed in amounts from 0.05 to 1%, preferably from 0.05 to 0.5% and, most preferably, from 0.3 to 0.7% by weight.

The amount of emulsifier and thickener employed will vary based on the identity of pH level to be achieved, the amounts of non-volatiles to be dispersed and the form of the final composition.

The non-volatiles in the compositions of the invention including the emollient, typically ranges from about 3 to 40% by weight, and preferably from about 5 to 30% by weight. The volatiles content is generally from about 55% to 97% by weight, preferably from about 65 to 96% by weight, and most preferably from 78 to 81% by weight for the preferred lotions and creams.

In general, the preferred emollient blend is employed as follows:

| Ingredient | Weight Percent |
|---|---|
| polyhydric alcohol | 0.5 to 10% |
| hydrocarbon | 1.0 to 15% |
| petrolatum | 0.1 to 1% |
| fatty acid ester | 0.5 to 5% |
| fatty acid | 0.5 to 3% |
| fatty alcohol | 1.0 to 6% |

In order to improve the lubricity of these and other personal care compositions and to provide emollience without a greasy feel, a water soluble silicone oil or fluid may be employed, such as a dimethyl polysiloxane, a methylphenyl polysiloxane and and an alcohol-soluble silicone glycol copolymer. Preferred siloxanes include dimethyl polysiloxane (CTFA name - dimethicone) - a polysiloxane end-blocked with trimethyl units and polydimethylcyclosiloxane, (CTFA name - cyclomethicone). The preferred siloxanes exhibit a viscosity from about 2 to 10 centistokes at 25°C. The siloxanes are employed in sufficient amounts to aid in stimulating removal of scale from dry skin and to aid in controlling conditioning, usually from about 0 to 10% by total weight of the composition, preferably from about 0.5 to 3%, and most preferably, from 0.2 to 1%.

Other conventional additives typically employed in cosmetic compositions may be employed. Fragrance oils, which mask the odor of the base and provide cosmetic appeal, can be employed, usually in amounts from 0.0005% to 2%. Nontoxic and compatible dyes may be utilized to color the composition, as desired. Preservatives, such as methylparaben or other esters of parahydroxy benzoic acid, or sodium dehydroacetate can be employed, generally in amounts from about 0.01% to 0.5%. If desired, formaldehyde and other similar preservatives can also be utilized.

In addition, other ingredients can be employed beneficially to provide a specifically tailored cosmetic composition. For example, a sun screen additive, such as salicylic acid derivatives, cinnamic acid derivatives,

benzophenone derivatives, coumarin derivatives, azoles, imidazole derivatives, naphtosulfonates, quinine salts and hydroquinone and its derivatives, especially benzylidene camphor, ethylhexyl para methoxy cinnamate, 5-benzoyl-4-hydroxy methoxy benzene sulfonic acid and octyl dimethyl para-aminobenzoic acid can be employed in the inventive composition in amounts from about 1% to 8% by weight of the total composition.

To provide a skin protectant composition, zinc oxide and like ingredients can be provided in amounts from about 0.5% to 3% by weight of the composition.

Essential oils, such as menthol, quaternary ammonium salts, soap solutions and the like can also be employed to provide formulations providing specific beneficial treatment. Additional materials that can be incorporated to provide therapeutic benefits are selected from over-the-counter drug active ingredients: hydrocortisone, hydrocortisone acetate, benzocaine, lidocaine, and methyl salicylate are examples of these.

The vehicle of the invention can be formulated into face creams, hand and body lotions, after shave lotions, shave creams, sunscreen creams, lotions and gels, shampoos, creme rinses, hydroalcoholic refresher-conditioner gels, aerosol sprays, liquid skin and scalp cleansers, stick deodorants and/or antiperspirants and others within the spectrum of personal care products.

The following formulations represent preferred embodiments of the invention when utilized as lotions or creams.

| Ingredient | Weight percent |
|---|---|
| amphoteric emulsifier | 0.20 - 0.60 |
| carboxy vinyl polymer | 0.10 - 0.30 |
| hydrocarbon oil | 1.00 - 12.00 |
| polyhydric alcohol | 0.50 - 5.00 |
| fatty alcohol | 1.00 - 6.00 |
| fatty acid ester | 0.50 - 5.00 |
| fatty acid | 0.50 - 3.00 |
| silicone oil | 0.20 - 1.00 |
| preservatives | 0.10 - 0.30 |
| fragrance | 0.05 - 0.15 |
| deionized water | 95.85 - 65.65 |
| | 100.00 100.00 |

The preferred lotion formulations typically exhibit a viscosity from about 2000 to 20,000 centipoises (cps) at ambient temperature. The preferred facial creams exhibit a viscosity from about 25,000 to 60,000 cps and the heavier creams or pastes show viscosities from 70,000 to 170,000 cps.

To prepare the composition of the invention, the emollients and other optional ingredients are charged and melted together using moderate agitation at temperatures of about 190°F. The amphoteric surfactant is then added to that charge at elevated temperatures. To the melted emollients and surfactant charge, water is slowly added under heat and moderate stirring to disperse the ingredients. The resulting hot dispersion is homogenized at using a homogenizing mixer as pressures from about 2000 to 2500 p.s.i.g. to form a milky emulsion. The appropriate quantity of a 1% solution of thickener is added and the resulting emulsion is cooled to room temperatures with agitation.

When employed as a facial product, a high viscosity mineral oil is preferably employed, (355 SUS) rather than petrolatum, which is preferably utilized in hand and body formulations in combination with a lower viscosity mineral oil (90 SUS).

The following Examples serve to illustrate certain preferred embodiments of the invention and are merely illustrative of its efficacy.

## EXAMPLE I

A facial lotion of the invention was prepared in accordance with the general preparation procedure having the following composition:

| Ingredients | Weight Percent |
| --- | --- |
| Disodium laurimino dipropionate | 0.35 |
| *Carbomer 934-carboxyl vinyl polymer | 0.10 |
| Stearic Acid | 1.50 |
| Isopropyl palmitate | 3.00 |
| Mineral Oil | 8.50 |
| Cetyl Alcohol | 3.00 |
| Propylene Glycol | 2.00 |
| Methyl p-hydroxybenzoic Acid | 0.10 |
| Propyl p-hydroxybenzoic Acid | 0.10 |
| Sodium dehydroacetate | 0.10 |
| Dimethyl polysiloxane | 0.50 |
| Fragrance | 0.05 |
| Deionized water | 80.70 |
| | 100.00 |

* Carbomer 934 is a trademark of B.F. Goodrich Co. for a cross-linked acrylic acid polymer having a Brookfield viscosity from 30,000 - 40,000.

A sample of the resulting facial lotion was photographed through a microscope at 2700X power and compared with similar photographs of samples of commercially available lotions including: Silkience Lotion; Clinique Lotion; Oil of Olay Lotion and Estee Lauder Lotion. The inventive facial lotion exhibited a more uniform dispersion having smaller sized particles. The pH of the lotion was 5.9 and its viscosity was 15,400 cps.

EXAMPLE II

A day cream of the invention was prepared having the same composition of Example I with the exception that the carboxyl vinyl polymer was present at 0.25 wt percent and the deionized water was present at 80.55 wt percent.

The pH of the day cream was 5.2 and its viscosity was 32,000 cps.

EXAMPLE III

In order to determine the effect on the properties of the inventive compositions imparted by the amphoteric emulsifier, cream formulations identical to that of Example I were prepared with the following modifications and tested for pH and viscosity as follows:

| Sample No. | Emulsifier (wt%) | Deionized Water (wt%) | pH | Viscosity (cps) |
|---|---|---|---|---|
| 1 | 0 | 80.85 | 4.60 | 15,800 |
| 2 | 0.10 | 80.75 | 4.85 | 42,500 |
| 3 | 0.25 | 80.60 | 5.35 | 47,500 |
| 4 | 0.50 | 80.35 | 5.85 | 36,700 |
| 5 | 0.75 | 80.10 | 6.05 | 25,800 |

The results show that the unexpected interaction between the amphoteric emulsifier and carboxy vinyl thickener serves to control pH and viscosity to maximize stability, to minimize skin irritation, and to create cosmetic elegance.

EXAMPLE IV

A hand and body lotion of the invention was prepared by charging to a first mixer, petrolatum, stearic acid, isopropyl palmitate, mineral oil, silicone fluid, cetyl alcohol and propyl paraben and heating the resulting emollient mix to 190°F under moderate agitation. A second mixer was charged with deionized water, disodium lauryl-ß-iminodipropionate, methyl paraben, sodium dehydroacetate, sorbitol, and dye. The second mixer contents were heated to 190°F under moderate agitation. Thereafter, the contents of the first mixer were added to the contents of the second mixer under moderate agitation. The resulting hot emulsion was homogenized at pressures from 2000 to 2500 psig. The homogenized

emulsion was cooled to from 80 - 85°F. To the cooled emulsion was added a solution of Carbolpol 934 and methyl paraben in deionized water. That solution was prepared by sifting Carbopol 934 into deionized water under rapid agitation; adding methyl paraben; heating to 150°F, agitating the ingredients until in solution and cooling to 80-85°F.

The resulting hand and body lotion had the following composition:

| Ingredient | Weight Percent |
|---|---|
| *Deriphat 160 (solution pH 12) | 0.60 |
| Carbopol 934 | 0.30 |
| Stearic Acid | 1.50 |
| Isopropyl palmitate | 2.50 |
| Petrolatum | 1.00 |
| Mineral Oil | 2.00 |
| Cetyl alcohol | 3.75 |
| Sorbitol | 3.85 |
| Silicone fluid | 0.75 |
| Methyl paraben | 0.10 |
| Propyl paraben | 0.10 |
| Sodium dehydroacetate | 0.10 |
| Fragrance | 0.10 |
| Deionized water | 83.35 |
| | 100.00 |

The hand and body lotion exhibited a pH of 5.7 and a viscosity between about 45,000 - 55,000.

---

* disodium lauryl-ß-iminodipropionate sold by General Mills/Henkel

EXAMPLE V

In order to illustrate the properties imparted to the inventive compositions by the amphoteric emulsifier, a series of compositions were prepared in which other anionic, cationic and amphoteric emulsifiers were compared to a preferred emulsifier of the invention.

A formulation was first prepared having the following composition:

| Ingredient | Weight Percent |
|---|---|
| Deriphat 160 | 0.15 |
| Carbopol 934 | 0.15 |
| Glycerine | 8.00 |
| Petrolatum | 6.00 |
| Mineral Oil | 2.00 |
| Cetyl alcohol | 2.00 |
| Stearic acid | 4.00 |
| Isopropyl palmitate | 2.00 |
| paraben(s) | 0.20 |
| Sodium dehydroacetate | 0.15 |
| Silicone fluid | 0.75 |
| Deionized water | 74.60 |
| | 100.00 |

Seven samples were prepared in the same way in which a different emulsifier was substituted for the Deriphat 160 in the amounts shown in Table 1 as follows:

TABLE 1

| Sample No. | Emulsifier | Amount |
|---|---|---|
| 1 | None | None |
| 2 | Deriphat 160 | 0.15 |
| 3 | [1]Gafac MC-470F | 0.16 |
| 4 | [2]Standapol ES-2 | 0.58 |
| 5 | [3]Barquat CT-429 | 0.52 |
| 6 | [4]Bioterge AS-40 | 0.38 |
| 7 | [5]Sodium lauryl sulfate | 0.15 |
| 8 | [6]Oleic Acid | 0.15 |
| 9 | [7]Cocoamido propyl betaine | 0.50 |

[1]Gafac MC-470F is sodium polyethylene glycol 200 lauryl ether phosphate.

[2]Standapol ES-2 is poly(oxy-1,2-ethanediyl-sulfo-omega-dodecyloxy)-sodium salt.

[3]Barquat CT-429 is cetyl trimethyl ammonium chloride.

[4]Bioterge AS-40 is sodium $C_{14}$ - $C_{16}$ olefin sulfonate

[5]Sodium lauryl sulfate is the sodium salt of the monodecyl ester of sulfuric acid.

[6]Oleic acid is 9-octadecenoic acid.

[7]Cocamidopropyl betaine is cocamidopropyl dimethyl glycine

The samples were tested for initial and 24 hour viscosity, initial and 24 hour pH, emulsion appearance and stability. To test stability, the samples were cycled for one day at: 104°F; ambient temperature; and 35°F. The number of days before the emulsion separated was recorded. The results of the tests are reported in Table 2 as follows:

TABLE 2

| Sample | Emulsifer | Initial Vis./ 24 hr. Vis (cps) | Initial pH/ 24 hr. pH | Appearance | Days Before Separation |
|---|---|---|---|---|---|
| 1 | None | 29,600/ | 5.55/ | | 01 |
| 2 | Deriphat 160 | 61,600/ 144,400 | 5.35/5.4 | Smooth | 20 |
| 3 | Gafac MC-470F | 559,400/ 330,000 | 5.0/5.15 | Grainy | 07 |
| 4 | Standapol ES-2 | 72,900/ 61,200 | 4.9/5.0 | Grainy | 16 |
| 5 | Barquat CT-429 | UNSTABLE IMMEDIATELY | | --- | --- |
| 6 | Bioterge AS-40 | 157,700/ 50,800 | 4.9/4.9 | Smooth | 16 |
| 7 | Sodium lauryl sulfate | 132,800/ 37,900 | 4.95/5.0 | Smooth | 16 |
| 8 | Oleic Acid 165,000 | 55,800/ | 4.95/5.0 | Grainy | 01 |
| 9 | Cocamido propyl betaine | 549,500 62,500 | 4.95/4.9 | Grainy | 08 |

0134483

The tests serve to demonstrate the superior texture and stability imparted to the formulations of the invention by the amphoteric emulsifier of the invention. In addition, pH control within the skin range is readily achieved at low levels of emulsifier.

While various preferred embodiments of the present invention have been illustrated by means of specific examples, it is understood that the present invention is in no way to be limited thereto.

## CLAIMS

Therefore, I claim:

1. An aqueous stable non-occlusive, personal care composition which comprises:
   (a) an emollient base;
   (b) a carboxyl vinyl polymer thickener in amounts sufficient to provide controlled topical application of said composition; and
   (c) an amphoteric emulsifier in amounts sufficient to stabilize the composition and to selectively control the pH of the composition at a level compatible with topical skin application.

2. The composition of Claim 1 wherein the emulsifier is an alkaline salt of a N-fatty aminodipropionate:

3. The composition of claim 2 where the emulsifier is an alkaline salt of a N-fatty iminopropionate.

4. The composition of claim 1 wherein the carbonyl vinyl thickener is a cross-linked acrylic polymer.

5. The composition of claim 1 wherein the emollient base is in amounts from about 5 to 30% by weight of said composition.

6. The composition of claim 1 wherein the thickener is an amounts from about 0.05 to 5% by weight of said composition.

7. The composition of claim 6 wherein the emulsifier is in amounts from about 0.1 to 1% by weight of said composition.